# EUROPEAN PATENT APPLICATION

(11) **EP 3 272 268 A1**
(43) Date of publication of application: **24.01.2018**
(21) Application number: 16817696.4
(22) Date of filing: 10.06.2016
(51) Int. Cl.: A61B 1/00, G02B 23/24

(54) **INSERTION APPARATUS AND ENDOSCOPE EQUIPPED WITH INSERTION APPARATUS**

(30) Priority: 30.06.2015 JP 2015131666
(71) Applicant: Olympus Corporation, Hachioji-shi Tokyo 192-8507 (JP)
(72) Inventor: YAMAYA, Koji, Hachioji-shi Tokyo 192-8507 (JP)
(74) Representative: Schicker, Silvia
(86) International application number: PCT/JP2016/067412
(87) International publication number: WO 2017/002587

(57) **Abstract**

In the insertion device, the entire traction wire coupled with the swing base in the distal end constituting section is covered with a tubular elastic member in a watertight manner, and the elastic member is fixed at a position that is away from the opening of the treatment tool channel hole toward the proximal side by a predetermined distance in the longitudinal axis direction. The traction wire is not exposed to the outside, a contaminated fluid and the like do not adhere thereto during treatment, and the wrinkle of the elastic member shrunk at the time of raising the swing base is accommodated in the space created at a predetermined distance.

## Description

### Field of the Invention

The present invention relates to an insertion device in which a raising base that changes a moving direction of a treatment tool is disposed on a distal side, and relates to an endoscope including the insertion device.

### Description of the Related Art

In general, a treatment tool have various functions. The treatment tool, for example a forceps, is inserted into a channel of an insertion section of an endoscope main body, and extends out from an opening that is opened at a distal side. Also, there is known a swing mechanism that changes, into a desired direction, a moving direction of a treatment tool that extends out from an opening provided in a part of a distal end of an insertion section on its peripheral surface side.

As a typical swing mechanism, a swing base for a treatment tool (or a raising base for a treatment tool) is known. The swing base is coupled to an operation lever provided on an operation section side via a traction wire and is raised by operating the operation lever, to thereby change the moving direction of the treatment tool, such as a forceps, into a desired direction.

Usually, when an insertion section is inserted into a body cavity, contaminated fluid, resected living tissue, and the like (hereinafter referred to as contaminated fluid and the like), intrude from an opening portion of a storage chamber in which the swing base is disposed, and adhere to a swing base main body and its peripheral portion. After finishing treatment, it is essential to wash and disinfect an endoscope to prevent infection. Since the swing base stored in the storage chamber and a drive unit of the traction wire have a complicated structure, cleaning work to completely remove unwanted substances requires time and effort. In particular, since the traction wire is made by weaving thin steel wires, it is necessary to make sure that dirt does not remain between the wires.

To address the foregoing, Patent Literature 1, Jpn. Pat. Appln. KOKAI Publication No. 08-056900, for example, has suggested an endoscope in which: a storage chamber of a raising base and a drive chamber of a traction wire are separated from each other by a wall so as to prevent the traction wire from being entangled with the swing base; and a driving force transmission member that transmits the traction force of the traction wire to the raising base through the wall is disposed. The patent literature is intended to prevent entanglement of wires. In separating the storage chamber of the raising base and the drive chamber of the traction wire by the wall, an O-ring is fitted to a raising base drive lever to prevent contaminated fluid and the like in the storage chamber of the raising base from entering the drive chamber, thereby achieving watertightness between the storage chamber of the raising base and the drive chamber. This makes it hard for contaminated fluid and the like to adhere to the traction wire.

Because the wall separating the drive chamber of the traction wire and the storage chamber of the raising base, the new raising base drive lever, and a watertight member are aligned in a radial direction of the insertion section in the aforementioned Patent Literature 1, in contrast to the conventional structure in which the traction wire is directly connected to the treatment raising base to raise it, the diameter of a distal end of the insertion section of the endoscope is increased. To necessitate the strength of the raising base drive lever and the thickness of the wall serving as a fulcrum in order to raise the treatment raising base also increases the diameter.

Accordingly, an object of the present invention is to provide: an insertion device that achieves a watertight structure for a traction wire without increasing an outer diameter of a distal portion, makes it easy to wash a treatment swing base, and facilitates movement of a treatment tool at a time of raising the swing base; and an endoscope including the insertion device.

### SUMMARY

According to an embodiment of the present invention, there is provided an insertion device characterized by comprising: an operating portion that is operable and provided to an insertion section inserted into a lumen; a long traction member that is connected to the operating portion and moves in a longitudinal axis direction of the insertion section, to operate the operating portion; a distal end constituting section that is provided on a distal side of the insertion section and comprises: a treatment tool channel hole that communicates with a treatment tool insertion tube disposed in the insertion section; and an introducing hole that is provided away from an opening of the treatment tool channel hole toward a proximal side of the insertion section and guides the traction member; and a tubular elastic member in which the traction member is disposed, one end being connected to the operating portion in a watertight manner so as to prevent the traction member connected to the operating portion from being exposed to an external part, the other end being connected to the introducing hole in a watertight manner.

### BRIEF DESCRIPTION OF DRAWINGS

FIG. 1 is a cross-sectional view showing a cross-section structure of a distal end constituting section of an insertion device according to a first embodiment, seen from the top.
FIG. 2 is a view showing an external configuration of the distal end constituting section of the insertion device without a distal end cover.
FIG. 3 is a cross-sectional view showing a cross-section structure of the distal end constituting section of the insertion device in which a swing base is laid down.
FIG. 4 is a cross-sectional view showing a cross-section structure of the distal end constituting section of the insertion device in which the swing base is raised.
FIG. 5 is a view showing a configuration example in which an endoscope is equipped with the insertion device.
FIG. 6 is a cross-sectional view showing a cross-section structure of a distal portion of the insertion device in which the swing base of the distal end constituting section of the insertion device is laid down.
FIG. 7 is a cross-sectional view showing a cross-section structure of the distal end constituting section of the insertion device in which the swing base is raised.
FIG. 8 is a cross-sectional view showing a cross-section structure of an elastic member.
FIG. 9 is a cross-sectional view showing a cross-section structure of a tube for a wire as a first modification.
FIG. 10 is a cross-sectional view showing a cross-section structure of a tube for a wire as a second modification.
FIG. 11A is a view showing a configuration example of an engaging unit and a swing base as a first modification of an operating portion.
FIG. 11B is a view showing a configuration example of an engaging unit and a swing base as a second modification of the operating portion.
FIG. 11C is a view showing a configuration example of an engaging unit and a swing base as a third modification of the operating portion.
FIG. 11D is a view showing a configuration example of an engaging unit and a swing base as a fourth modification of the operating portion.
FIG. 12 is a view showing a configuration example of the operating portion as a first modification of a wire traction mechanism.
FIG. 13 is a view illustrating a first procedure of installing the engaging unit.
FIG. 14 is a view illustrating a second procedure of installing the engaging unit.
FIG. 15 is a view describing a configuration example according to a second modification of the wire traction mechanism.
FIG. 16 is a view describing a configuration example according to a third modification of the wire traction mechanism.
FIG. 17 is a view describing a configuration example according to a fourth modification of the wire traction mechanism.
FIG. 18 is a view describing a configuration example according to a fifth modification of the wire traction mechanism.
FIG. 19A is an external view of a retaining plate, seen from the top, which is a guide portion for fixing the tube for a wire and a coupling unit.
FIG. 19B is an external view of the retaining plate seen from the front.
FIG. 20 is a view illustrating a procedure of assembling the wire traction mechanism.
FIG. 21 is a cross-sectional view showing a cross-section structure of the swing base of the distal end constituting section according to a third embodiment.
FIG. 22 is a view of a configuration, seen from the lateral side, in which a cover is provided to the distal end constituting section of the insertion device.
FIG. 23 is a view of a configuration, seen from the distal side, in which the cover is provided to the distal end constituting section of the insertion device.

### DETAILED DESCRIPTION

Embodiments of the present invention will be described in detail with reference to the drawings.

### [First Embodiment]

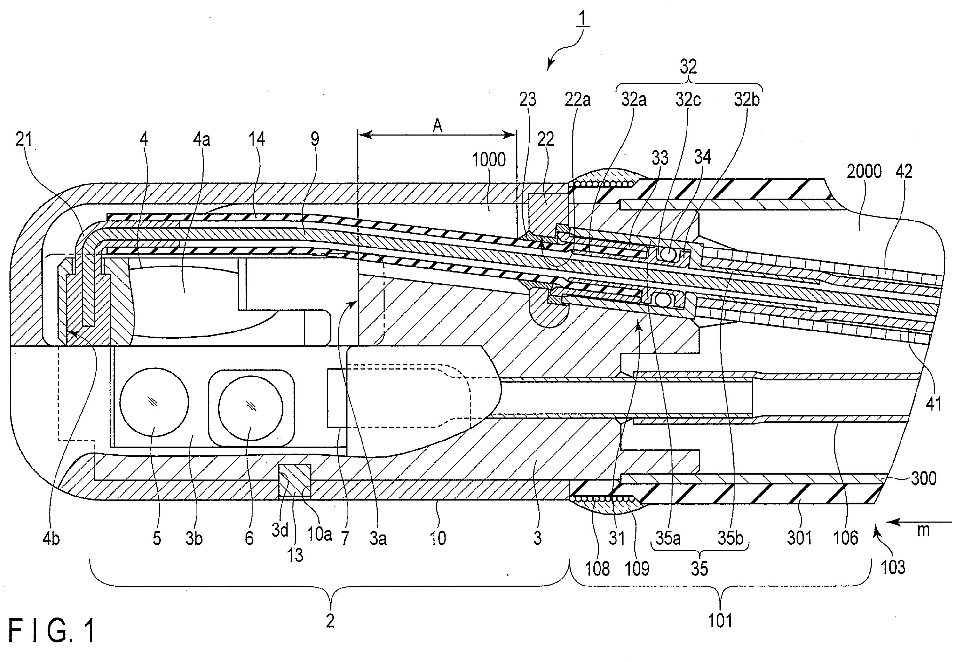

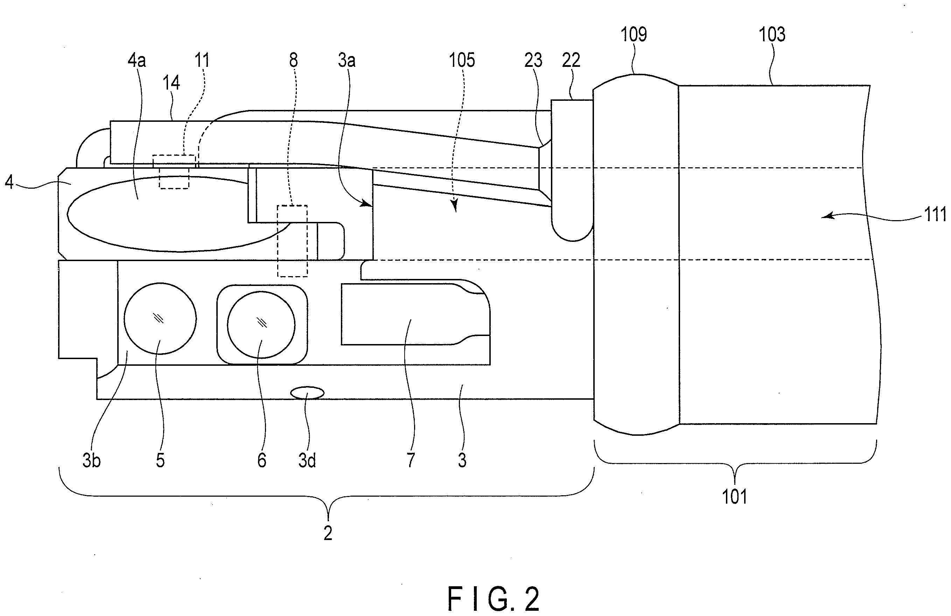

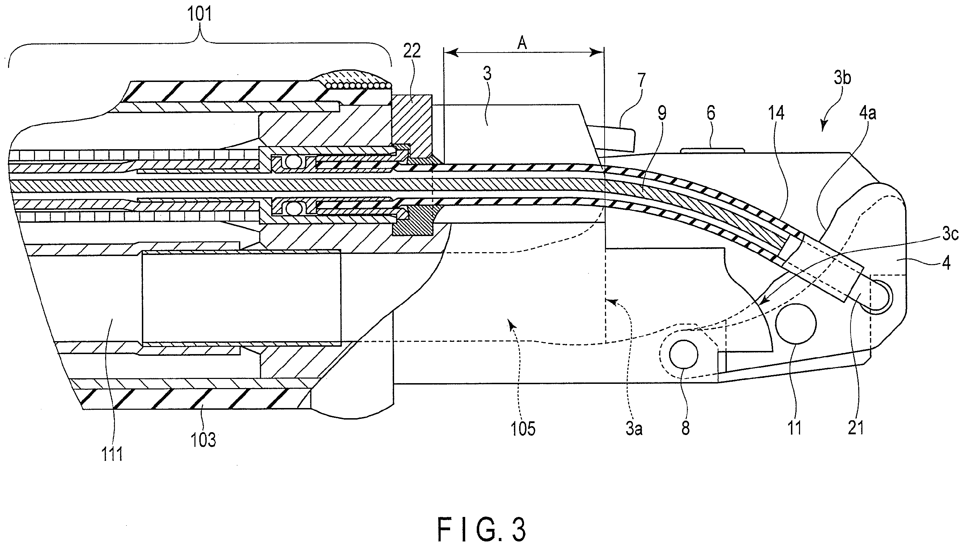

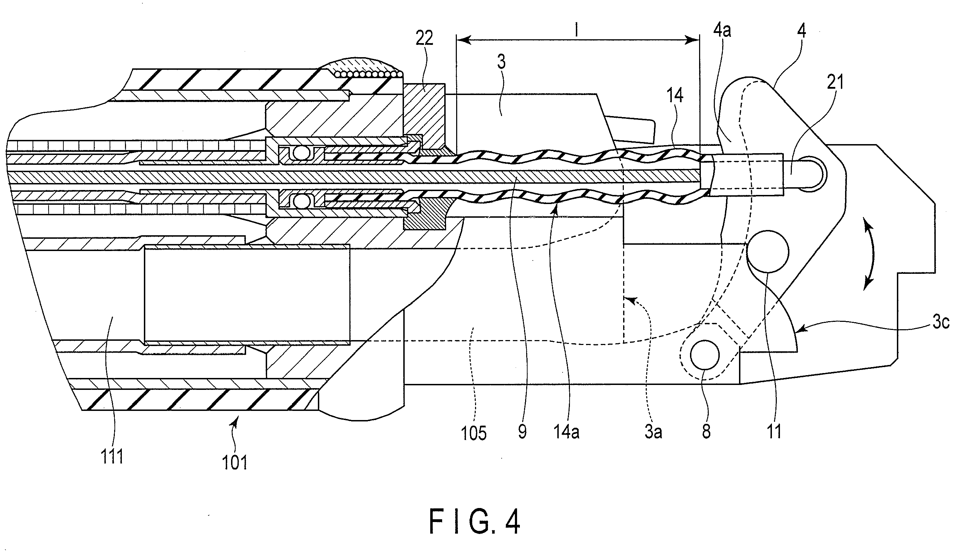

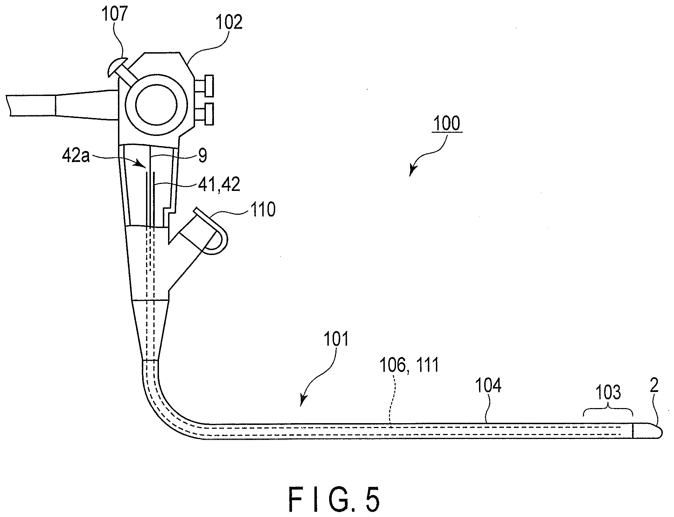

FIG. 1 is a cross-sectional view showing a cross-section structure of a distal end constituting section of an insertion device according to a first embodiment, seen from the top. FIG. 2 is a view showing an external configuration of the distal end constituting section of the insertion device without a distal end cover. FIG. 3 is a cross-sectional view showing a cross-section structure of the distal end constituting section of the insertion device in which a swing base is laid down. FIG. 4 is a cross-sectional view showing a cross-section structure of the distal end constituting section of the insertion device in which the swing base is raised. FIG. 5 is a view showing a configuration example in which an endoscope is equipped with the insertion device.

In the descriptions provided below, a swing mechanism that changes a moving direction (insertion direction) of the insertion device to a vertical direction (X-axis direction) when it is a horizontal axis direction (Y-axis), is employed as a swing base for a treatment tool (hereinafter referred to as a swing base) . This swing base may be referred to as a raising base. This swing base may be configured as a swing mechanism that is capable of changing the direction not only to the vertical direction but also to a left and right direction crossing the vertical direction. In each of the embodiments described below, only a swing mechanism that changes the direction to the vertical direction is shown to make the description simple. A mechanism that changes the direction to a left and right direction can be implemented by, for example, separating a portion supporting an axis of the swing base so that it turns in the vertical direction from a base member 3 described below, supporting the portion in a manner to turn around a horizontal axis, coupling the swing base with a traction wire having technical features described below, and arranging two vertical and horizontal traction wires in parallel in an insertion device. Needless to say, the aforementioned mechanism that changes the direction to a left and right direction is an example, and the mechanism is not limited to this structure. The mechanism may have a publicly-known structure.

First, a configuration example in which an endoscope is equipped with an insertion device 1 will be described.

The endoscope 100 is constituted by an insertion section 101 that is inserted into a lumen and an operation section 102. An insertion port (forceps port) 110 of a treatment tool is provided on a proximal side of the insertion section 101. A distal end constituting section 2 having a cylindrical shape is disposed on a distal side of the insertion section 101, and a swing base operation section 107 is provided to the operation section 102. This endoscope includes a general system structure (not shown) including, for example, a light source device that supplies illumination light, a control section including a video processor that performs image processing on an imaged video signal, a display section that displays an observation image,
and a recording section that records the video signal.

In the insertion section 101 includes following the distal end constituting section 2 to a bending portion 103 and a flexible tube 104, disposed toward the proximal side thereof. The insertion section 101 includes a channel conduit 111 for inserting a treatment tool, an air/water supplying tube 106 for supplying the air or a washing liquid, and a long traction wire 9 for causing a swing base 4 to swing. The insertion section 101 is connected to the swing base operation section 107 via the traction wire 9. By a lever operation of the swing base operation section 107, the swing base 4 swings to be raised or laid down. Among these elements, the swing base 4, an engaging section 21, the traction wire 9 and the swing base operation section 107 form a wire traction mechanism. Also, the swing base 4 and the engaging section 21 form an operating portion that is operable. The operation portion includes the swing base 4 that swings around a predetermined raising shaft 8 provided to the distal end constituting section 2 by the traction force of the traction wire 9, and that changes a direction of a treatment tool (not shown) protruding from a channel opening 3a of a treatment tool channel hole 105.

The channel conduit 111 passes through the insertion section 101 from the insertion port 110 on the proximal side and is connected to the treatment tool channel hole 105, and the channel opening 3a opens at the distal end. A treatment tool is inserted from the insertion port 110, passes through the channel conduit 111, and extends out from the channel opening 3a of the treatment tool channel hole 105.

The distal end constituting section 2 is generally constituted by a base member 3 formed of a hard material such as a metallic material, the swing base 4 turnably provided to the base member 3, and a distal end cover 10 having a cylindrical shape. The base member 3 and a first bending piece 300 on the most distal side of the bending portion 103 are fixed to each other by an adhesive, a screw, or the like. The bending portion 103 is covered with a covering material 301. A distal end of the covering material 301 is wrapped with a fixing thin wire 108 , and a surface layer side thereof is applied with an adhesive 109.

The base member 3 is divided into two sides in a longitudinal axis direction m: on one side, the channel opening 3a and the swing base 4 before the channel opening 3a are disposed; and on the other side, a flat observation surface 3b is formed with respect to the outer peripheral surface. The channel opening 3a communicates with the channel conduit of the insertion section 101 through the treatment tool channel hole 105. Also, an illumination window portion 5 to be irradiated with illumination light and an observation window portion 6 including an unshown imaging element therein are arranged on the observation surface 3b. A nozzle portion 7 is disposed in the vicinity of the observation window portion 6 and on the proximal side in the longitudinal axis direction m, and ejects a washing liquid such as saline, or a gas such as the air, supplied from the air/water supplying tube 106, to suitably wash the illumination window portion 5 and the observation window portion 6.

The distal end cover 10 is formed separately from the base member 3. The distal end cover 10 forms a shape of a cup that is injection-molded by a resin material or the like, and has an opening window, a part of which facing the observation surface 3b and the swing base 4 is opened. When fitted into the distal end constituting section 2, the distal end cover 10 has a cover hole 10a formed at a position opposite to a cover fastening hole 3d formed on the base member 3. By inserting a fixing portion 13 into the cover fastening hole 3d overlapped with the cover hole 10a, the distal end cover 10 is fixed to the distal end constituting section 2. The fixing portion 13 may have a screw clamp structure by forming a screw groove the cover fastening hole 3d.

The swing base 4 has a concave shape, has an abutting surface 4a that abuts on a treatment tool extending out from the channel opening 3a to change its moving direction. An inner surface of the swing base 4 facing the base member 3 engages with the raising shaft 8 (supporting portion) provided to the base member 3, to be turnably supported; and a guide pin 11 is provided in a substantially central area of the swing base 4. Also, the engaging section 21 is provided on the outer surface of the swing base 4, and the traction wire 9 as a traction member is connected thereto. The guide pin 11 moves above a guide surface 3c on a distal side of the base member 3 ; and at a point where the guide pin 11 abuts on the guide surface 3c, the raising stops, as shown in FIG. 4.

The swing base 4, the engaging section 21, and the traction wire 9 of the wire traction mechanism will be described.

A distal end of the traction wire 9 is inserted into the cylindrical engaging section 21 to be fixed. The traction wire 9 is covered with a tube 14 for the wire that is made of an elastic member having a tubular shape with a diameter that secures a certain clearance without sticking to the traction wire 9. One end of the tube 14 for the wire is inserted into a small-diameter part of the engaging section 21 and adhered thereto so as to be watertight; and the other end is connected to one end of a coupling unit 31. The other end of the coupling unit 31 is coupled with a guide tube 41 (introducing hole) for the wire that covers the traction wire 9 inserted through the insertion section 101 and a wire guide member 42 that covers it. In this embodiment, the tube 14 for the wire ranging from the engaging section 21 to the coupling unit 31 is installed in its natural length without expansion or contraction when the swing base 4 is laid down, as shown in FIG. 3. However, it does not always have to be installed in its natural length, but may be installed in an expanded manner when the swing base 4 is laid down, within the range that does not inhibit the raising operation by its elastic force.

The coupling unit 31 comprises: a coupling member 32 including an insertion portion 32a inserted into the tube 14 for the wire and a U-groove 32b; an engaging member 33 having a tubular shape that is externally arranged on the inserted part of the tube 14 for the wire, to press and fix it; an O-ring 34 that is fitted to the U-groove 32b; and a cover member 35 including a pressing portion 35a, which presses the O-ring 34 from the outer peripheral side, and an insertion portion 35b inserted into the guide tube 41 for the wire.

The cover member 35 presses the O-ring 34 from the outer peripheral side, and separates an inner part of the tube 14 for the wire from an outer part 1000 of the insertion section so as to be watertight. Moreover, the insertion portion 35b is fixed by an adhesive or the like in a state where it is inserted into the guide tube 41 for the wire. In the case of simplifying the structure of the coupling unit 31, it is possible to adhere each of the coupling member 32 and the engaging member 33 to the cover member 35 without using the O-ring 34, to achieve watertightness.

The tube 14 for the wire and the coupling unit 31 are inserted into a cutout portion 22a of a retaining plate 22, and adhered thereto so that the outer peripheral surfaces of the tube 14 for the wire and the coupling unit 31 and the cutout portion 22a will be watertight.This adhesion is provided by filling a silicone-based adhesive 23, for example, in a gap among the tube 14 for the wire, the coupling unit 31 and the cutout portion 22a. The tube 14 for the wire is not particularly limited as long as it is made of a material that is at least safe for a human body, easy to compress (or expand) in its longitudinal direction, and strong against repeated compression, but a urethane tube or a fluorine rubber, for example, is preferred. More preferably, the tube 14 for the wire is a urethane tube having a fluorine-based resin coating on the outer surface thereof.

The cutout portion 22a of the retaining plate 22 that guides the traction wire 9 is provided at a position that is away from the channel opening 3a toward the proximal side by the distance A in the longitudinal axis direction, as shown in FIGS. 1 and 3. In this embodiment, since the adhesive 23 is formed in a manner protruding from the cutout portion 22a, the distance A is specifically a distance from the channel opening 3a to a distal portion (a position where the tube for the wire is fixed) of the adhesive 23 of the cutout portion 22a. Namely, this distance A is used as a space for accommodating a wrinkle 14a of the tube 14 for the wire that is shrunk when the swing base 4 is raised, as shown in FIG. 4.

That is, when the tube 14 for the wire is shrunk to the distance I, the wrinkle 14a of the tube 14 can be formed not only on the distal side of the channel opening 3a, but also between the channel opening 3a and the cutout portion 22a of the retaining plate 22. Accordingly, if the swing base 4 is in a raised state, it is possible to reduce the amount of deformation of the whole tube 14 for the wire caused at the time of shrinkage, relative to the entire length, and reduce the height (amplitude) of the wrinkle 14a or the number of wrinkles if the height is the same. Since the adhesive 23 is not always formed in a manner protruding from the cutout portion 22a as described in this embodiment, the distance A is set to a distance from the position of the channel opening 3a to the position where the tube for the wire is fixed.

Therefore, the position where the tube for the wire is fixed (an end position on the proximal side where the tube 14 for the wire can be deformed) may not only be the distal position of the adhesive, but also the position of the cutout portion 22a of the retaining plate 22 or a position where the tube is fixed by another component.

This distance A, that is, the space for accommodating the wrinkle 14a of the tube 14 for the wire is set according to the traction amount (distance) of the traction wire 9 and the elastic deformation of the tube 14 for the wire. This is set at least in a way that prevents the wrinkles 14a generated in the tube 14 for the wire from sticking to each other when the swing base 4 is pulled by the traction wire 9 to be raised and the tube 14 for the wire is shrunk to the distance I, as shown in FIG. 4.

By including not only the distal side of the channel opening 3a, but also the distance A between the channel opening 3a and the cutout portion 22a of the retaining plate 22 or the adhesive 23 for the tube 14 for the wire in this manner when the swing base 4 is laid down, it is possible to reduce the size and the number of wrinkles 14a when the tube 14 is shrunk to the distance I. Namely, the longer the distance I is set, the smaller the height (amplitude) of the wrinkle 14a will be, allowing reduction of the size and the number of wrinkles 14a. In this embodiment, the distance A is obtained by moving the retaining plate 22, which fixes the tube 14 for the wire, closer to the proximal side than the channel opening 3a relative to the swing base 4 when arranging each component at the time of design. This movement merely increases the distance in the longitudinal axis direction for the base member 3, and is realized without increasing the outer diameter of the distal end constituting section 2. Also, since the distal end constituting section 2 is used in a state where the distal end cover 10 is fitted thereto, no problem occurs in raising and laying down the swing base even if the distance A part in which the tube 14 for the wire moves increases.

The distal end constituting section 2 of the insertion device of this embodiment described above includes an end portion of the traction wire 9, and the entire traction wire 9 is covered with the tube 14 for the wire made of a tubular elastic member in a watertight manner, so that the traction wire 9 is not exposed to the outside. Therefore, even if a contaminated fluid and the like remain in the periphery of the swing base 4 or in the vicinity of the channel opening during treatment, it will not touch the traction wire 9.

In addition, the height (amplitude) of the wrinkle 14a generated in the tube 14 for the wire at the time of raising the swing base is reduced, and even if a treatment tool is moved forward and backward in the vicinity, the treatment tool does not interfere with the wrinkle 14a. Also, since the distance I at the time of raising the swing base 4 has a certain length relative to the natural length, a load imposed by expansion and contraction of the elastic member is reduced, and even if the action of raising and laying down is repeated, the tube does not degrade, leading to improvement of the durability.

Since both the size and the number of wrinkles 14a caused at the time of raising can be reduced, even if washing and disinfection are performed while the swing base 4 is raised, a sufficient washing effect can be achieved because there are no overlapping portions of the wrinkle 14. Also, since the tube 14 for the wire and the guide tube 41 for the wire are coupled with each other by the coupling unit 31 with a watertight structure, even if the tube 14 for the wire is incidentally damaged, and polluted water intrudes into the cover from the outside, the polluted water does not directly enter an insertion section internal part 2000. An end portion of the guide tube 41 for the wire of this embodiment is opened in the operation section 102, as shown in FIG. 5.

### [Second Embodiment]

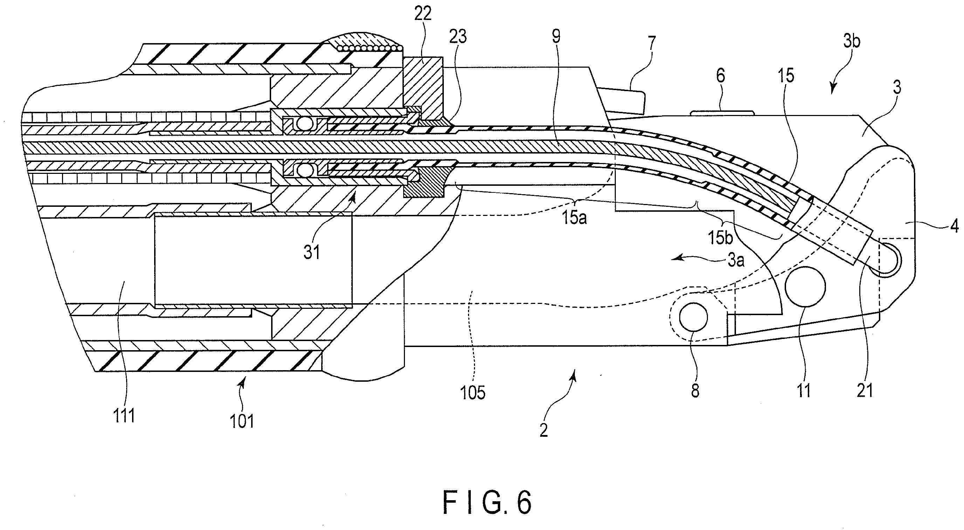

A second embodiment will be described with reference to FIGS. 6 to 8.

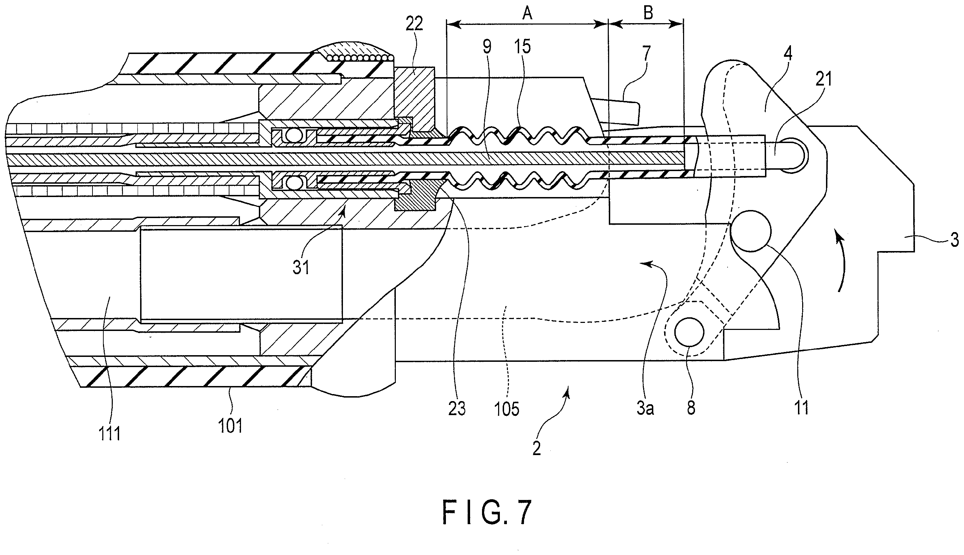

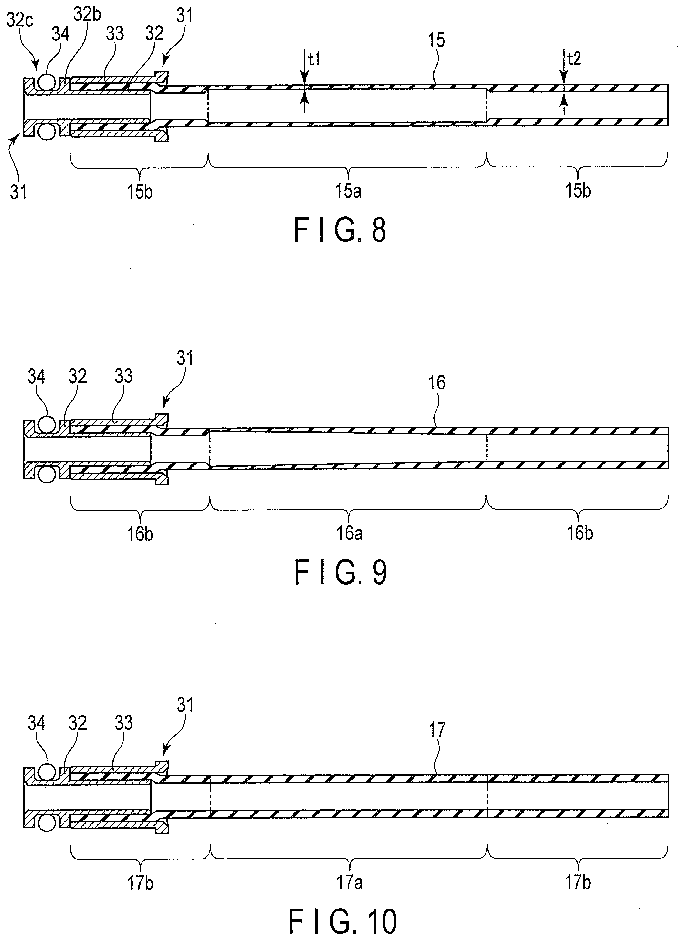

FIG. 6 is a cross-sectional view showing a cross-section structure of a distal portion of the insertion device in which the swing base of the distal end constituting section of the insertion device is laid down. FIG. 7 is a cross-sectional view showing a cross-section structure of the distal end constituting section of the insertion device in which the swing base is raised. FIG. 8 is a cross-sectional view showing a cross-section structure of the tube for the wire. In the description of this embodiment, the structural parts equivalent to those of the first embodiment are identified by the same reference symbols as those used for the first embodiment, and detailed explanations thereof are omitted.

The distal end constituting section 2 of the insertion device of this embodiment is different from that of the first embodiment in the cross-sectional shape of the tube for the wire, and is the same as that of the first embodiment in the other structural parts. A tube 15 for the wire shown in FIG. 8 is formed of the same elastic material as that of the tube 14 for the wire of the first embodiment, and has parts that are different from each other in thickness. Usually, if an elastic member having two different thicknesses is shrunk in a longitudinal axis direction, a thinner part of the elastic member will have a wrinkle first. The tube 15 for the wire of this embodiment is configured so that areas 15b, which respectively range from the vicinity of the parts coupled with the engaging section 21 and the coupling unit 31 on both sides of the cover to the end parts, have a thickness t2 that is greater than a thickness t1 of an area 15a in the middle where it is desired to form a wrinkle (t1<t2).

The tube 15 for the wire is installed in its natural length in a state where the swing base 4 is laid down, as shown in FIG. 6. If the swing base 4 is raised from this state by pulling the traction wire 9, a wrinkle is actively generated in the thinner area A (15a) having the thickness t1, as shown in FIG. 7.

In this manner, when the tube 15 for the wire shrinks, it can have the area A in which a wrinkle is actively generated and an area B in which it is desired to inhibit generation of a wrinkle. In this embodiment, when the swing base 4 is raised, generation of a wrinkle is inhibited in the area B in which a treatment tool that moves forward and backward intersects with the traction wire 9, and a wrinkle is actively generated in the area A that does not cross other structural parts including the treatment tool, as shown in FIG. 7.

As described above, according to this embodiment, the position of the wrinkle generated at the time of raising the swing base 4 can be set as one chooses, by adjusting the thickness of the tube 15 for the wire. Thereby, the tube 15 for the wire can avoid generation of a wrinkle in the area close to a treatment tool that is moved forward and backward.

### [First Modification of Tube for Wire]

A first modification of the tube for the wire will be described.

FIG. 9 is a cross-sectional view showing a cross-section structure of a tube for a wire as a first modification. In the description of this modification, the structural parts equivalent to those of the first embodiment are identified by the same reference symbols as those used for the first embodiment, and detailed explanations thereof are omitted.

The tube 16 for the wire of this modification is formed of the same material as that of the tube 15 for the wire described above, and includes a tapered area 16a in which an outer diameter is constant and an inner diameter changes to be tapered, and a constant thickness area 16b in which the thickness is constant. The tapered area 16a starts from the coupling unit 31 on the proximal side to the engaging section 21 of the swing base 4 not shown, and is tapered so that an inner diameter is linearly decreased.

According to this modification, the tube 16 for the wire is configured so that a wrinkle with the largest amplitude is generated at the thinnest part of the tapered area 16a when the swing base 4 is raised, and that the amplitude of the wrinkle gradually decreases as the thickness increases. Accordingly, it is possible to prevent a wrinkle generated at the time of raising the swing base 4 from interfering with an inserted treatment tool by decreasing the size of the wrinkle on the swing base 4 side and increasing the size of the wrinkle on the coupling unit 31 side.

### [Second Modification of Tube for Wire]

A second modification of the tube for the wire will be described.

FIG. 10 is a cross-sectional view showing a cross-section structure of a tube for a wire as a second modification. In the description of this modification, the structural parts equivalent to those of the first embodiment are identified by the same reference symbols as those used for the first embodiment, and detailed explanations thereof are omitted.

A tube 17 for a wire of this modification is formed of the same material as that of the tube 14 for the wire described above, and is configured to have an area in which a wrinkle is generated by partially changing the hardness. By the publicly known technique, the middle area 17a of the tube 17 for the wire shown in FIG. 10 is formed as a soft part, and the side areas 17b on both sides of the middle area 17a shown in FIG. 10 are formed to be harder than the middle area 17a. The difference in hardness can be realized by, for example, integrally forming the tube using the same main ingredient and selecting different degrees of hardness.

According to this modification, when the swing base 4 is raised, the tube 17 for the wire can have a large wrinkle actively generated in the soft middle area 17a, and can be prevented from having a wrinkle in the vicinity of the coupling unit 31 and the engaging section 21. Accordingly, it is possible to inhibit generation of a wrinkle on the swing base 4 side and prevent a wrinkle generated at the time of raising the swing base 4 from interfering with an inserted treatment tool.

### [First Modification of Operating Portion]

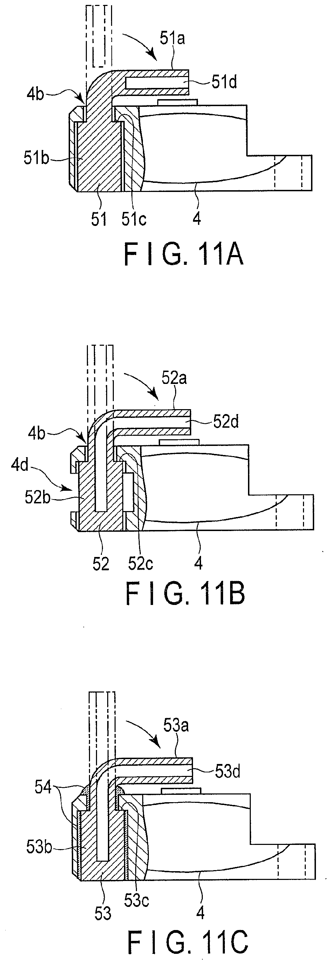

A configuration example of the engaging unit and the swing base as a first modification of the operating portion will be described with reference to FIG. 11A.

An engaging unit 51, which has a cylindrical shape, comprises a small-diameter part 51a and a large-diameter part 51b with a step (large-diameter part) 51c. The small-diameter part 51a is extended out from an engaging hole 4b of the swing base 4, and is bent at a substantially right angle. The engaging unit 51 is fitted into the engaging hole 4b with a clearance. Since the inner diameter of the engaging hole 4b of the swing base 4 is smaller than the outer diameter of the large-diameter part 51c, the engaging unit 51 is supported turnably without dropping out of the engaging hole 4b.

The engaging unit 51 shown in FIG. 11A has a fixing hole 51d formed at a depth from the distal surface of the small-diameter part 51a to the front of the bent part. A distal end of the traction wire 9 is inserted into this fixing hole 51d, as described below, and is fixed thereto by brazing or soldering, etc., which provides strong fixation.

### [Second Modification of Operating Portion]

A configuration example of the engaging unit and the swing base as a second modification of the operating portion will be described with reference to FIG. 11B.

An engaging unit 52, like the above-described engaging unit 51, has a cylindrical shape and comprises a small-diameter part 52a and a large-diameter part 52b with a step 52c. The engaging unit 52 is provided so that the small-diameter part 52a is extended out from the engaging hole 4b of the swing base 4 and bent at an approximate right angle. A fixing hole 52d is formed at a depth from the distal surface of the small-diameter part 52a to the middle of the large-diameter part 52b.

In this modification, a window 4d, where the lateral side of the engaging hole 4b of the swing base 4 is partially opened, is formed, and the side surface of the fitted large-diameter part 52b is partially exposed.

Since the large-diameter part is partially exposed to the outside in this modification, in addition to the clearance provided between the engaging hole 4b and the large-diameter part 52b, it is possible to efficiently pour a chemical to wash the swing base 4.

### [Third Modification of Operating Portion]

A configuration example of the engaging unit and the swing base as the third modification of the operating portion will be described with reference to FIG. 11C.

An engaging unit 53, like the above-described engaging unit 51, has a cylindrical shape and comprises a small-diameter part 53a and a large-diameter part 53b with a step 53c. The engaging unit 53 is provided so that the small-diameter part 53a is extended out from the engaging hole 4b of the swing base 4 and bent at a substantially right angle. A fixing hole 53d is formed at a depth from the distal surface of the small-diameter part 53a to the middle of the large-diameter part 53b.

In this modification, an adhesive 54 is filled in a clearance between the engaging hole 4b and the engaging unit 53 to fix the engaging unit 53 to the engaging hole 4b. Namely, the clearance to be washed is removed so that there is no gap for a contaminated fluid to enter. Even if the engaging unit 53 and the swing base 4 are in a fixed state, there will be no practical problem on the action of raising and lying down as long as the angle at which the swing base is raised from the state of being laid down (0°) is not greater than 90°.

### [Fourth Modification of Operating Portion]

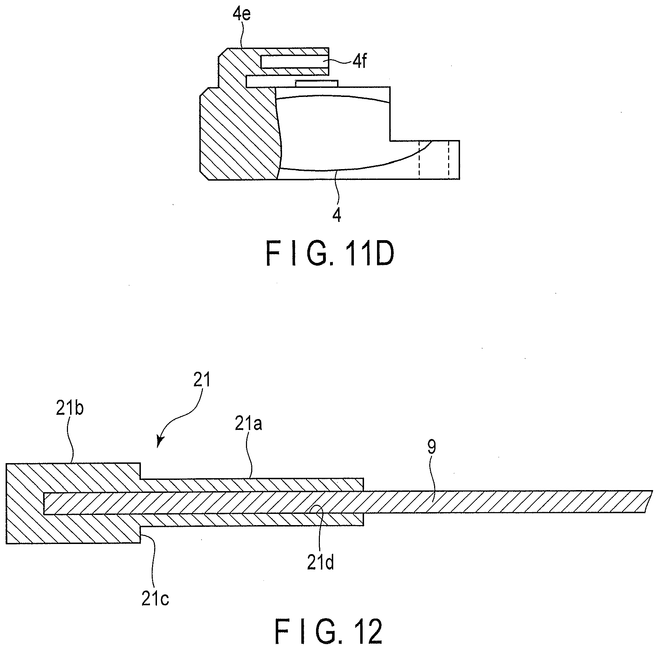

A configuration example of the swing base having a function of engaging with the traction wire, which is a fourth modification of the operating portion, will be described with reference to FIG. 11D.

In this modification, the swing base 4 is not engaged with a separate engaging unit, but includes an L-shaped engaging unit 4e provided integrally as a part of the swing base 4. A linear fixing hole 4f is formed at a depth similar to that shown in FIG. 11A from an end surface of the engaging unit 4e, and the traction wire 9 is inserted into the hole to be fixed.

### [First Modification of Wire Traction Mechanism]

A configuration example of the operating portion by the traction wire 9 and the engaging section 21 will be described as a first modification of the wire traction mechanism, with reference to FIG. 12.

The engaging section 21 comprises a small-diameter part 21a and a large-diameter part 21b with a step 21c. A fixing hole 21d is formed from the distal surface of the small-diameter part 21a to the middle of the large-diameter part 21b, and the distal end of the traction wire 9 is inserted into the fixing hole 21d to be fixed by an adhesive or soldering. Since the distal end of the traction wire 9 is inserted into the fixing hole 21d in this manner, the end portion of the wire is not exposed to the outside, either. In the case of fixing the traction wire 9 to the fixing hole 21d, the small-diameter part 21a of the engaging section 21 may be swaged to be fixed without using an adhesive.

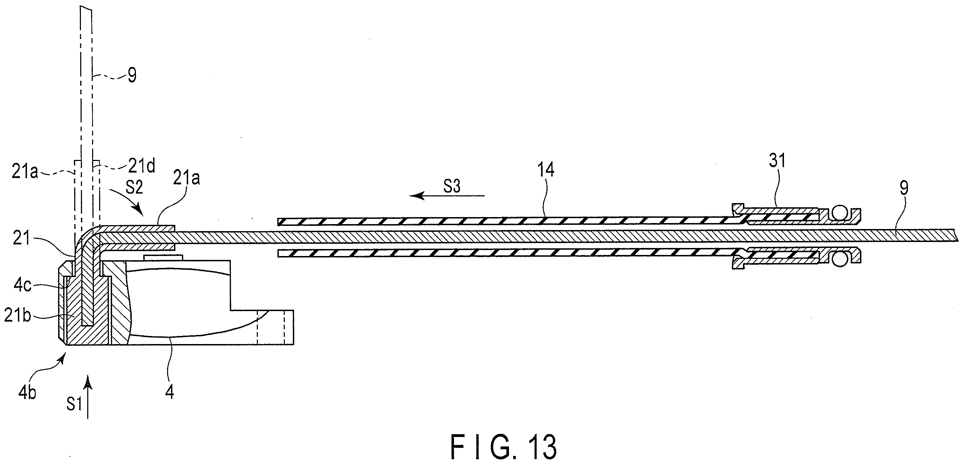

A first procedure of attaching the engaging section 21 to the swing base 4 will be described with reference to FIG. 13. First, the traction wire 9 is inserted into the fixing hole 21d of the small-diameter part 21a from the distal end of the traction wire 9, to be fixed by an adhesive (S1). After the unit is inserted into the swing base 4 in the direction of S1, the small-diameter part 21a is bent at a substantially right angle together with the traction wire 9 (S2).

Next, the distal end of the tube 14 for the wire, the proximal end of which is attached with the coupling unit 31, is inserted from the back end of the traction wire 9 in a manner to cover the traction wire 9 (S3). The distal end of the tube 14 for the wire is inserted until it covers the small-diameter part 21a of the engaging section 21, so that the tube 14 for the wire is fixed to the small-diameter part 21a by an adhesive.

Thereby, the traction wire 9 is entirely covered with the engaging section 21 and the tube 14 for the wire, and has no part that is exposed. Also, since the small-diameter part 21a is bent at an approximate right angle together with the traction wire 9, the traction wire 9 can be coupled with the swing base 4 in a manner that the swing base can turn freely. By the clearance between the engaging section 21 and the engaging hole 4b of the swing base 4 created by the turning, a washing liquid or a disinfectant, etc., circulates well at the time of reprocessing after finishing an examination.

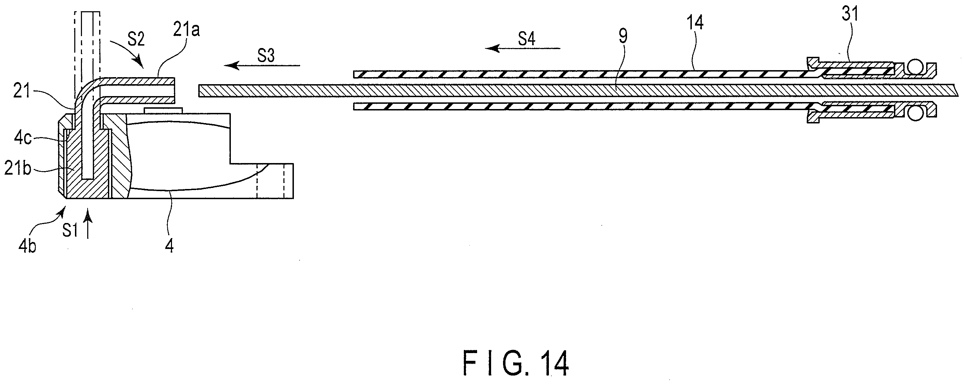

A second procedure of attaching the engaging section 21 to the swing base 4 will be described with reference to FIG. 14. First, the engaging section 21 is inserted into the engaging hole 4b of the swing base 4, and the small-diameter part 21a is extended out from the engaging hole 4b (S1). The small-diameter part 21a is bent backward at an approximate right angle (S2) . The traction wire 9 is inserted into the fixing hole 21d of the bent small-diameter part 21a from the distal end of the traction wire 9, and is fixed by an adhesive (S3).

Next, the distal end of the tube 14 for the wire, the proximal end of which is attached with the coupling unit 31, is inserted from the back end of the traction wire 9 in a manner to cover the traction wire 9. The distal end of the tube 14 for the wire is inserted until it covers the small-diameter part 21a of the engaging section 21, to be fixed by an adhesive (S4). Thereby, the traction wire 9 is entirely covered with the engaging section 21 and the tube 14 for the wire, and has no part that is exposed. In this example as well, by the clearance between the engaging section 21 and the engaging hole 4b of the swing base 4 created by the turning, a washing liquid or a disinfectant, etc. , circulates well at the time of reprocessing after finishing an examination.

### [Second Modification of Wire Traction Mechanism]

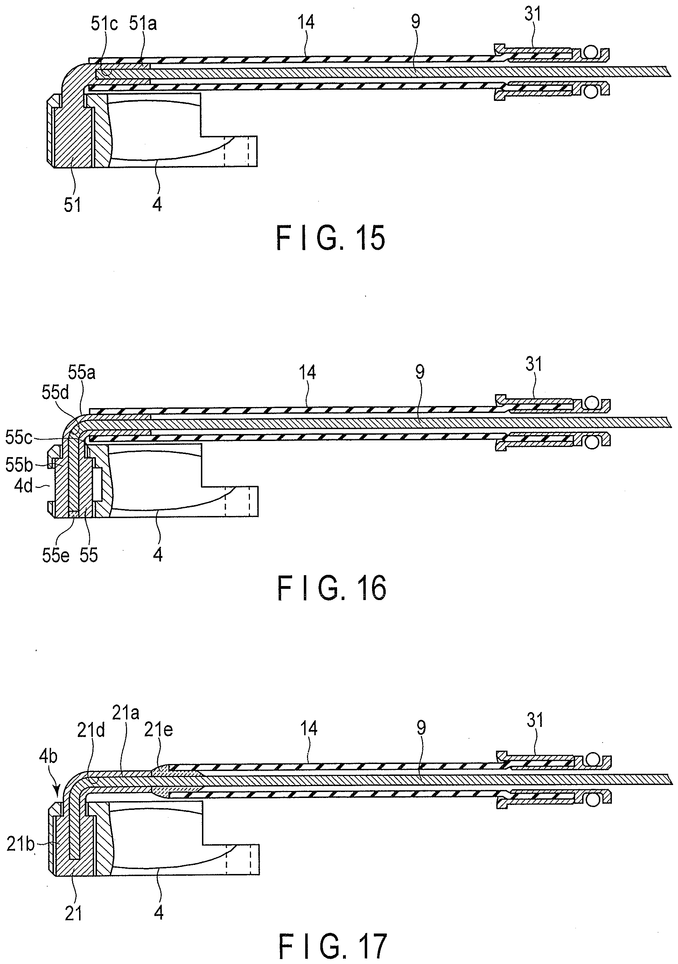

A configuration example by the traction wire 9 and the engaging unit 51 will be described as a second modification of the wire traction mechanism, with reference to FIG. 15. The components can be assembled by following the above-described second procedure shown in FIG. 14.

First, the engaging unit 51 is inserted into the engaging hole 4b of the swing base 4, and the small-diameter part 51a extended out is bent backward at an approximate right angle. The traction wire 9 is inserted into the fixing hole 51d of the bent small-diameter part 51a from the distal end of the traction wire 9, and is fixed by a strong fixing method such as brazing. Then, the distal end of the tube 14 for the wire, the proximal end of which is attached with the coupling unit 31, is inserted from the back end of the traction wire 9, to cover the small-diameter part 51a. At this time, the tube 14 for the wire and the small-diameter part 51a are fixed to each other by an adhesive.

With this configuration, the traction wire 9 connected to the operating portion can be prevented from being exposed to the distal end cover 10 by the tube 14 for the wire, and even if polluted water and the like enter the distal end cover 10 during treatment, the polluted water and the like will not adhere to the traction wire 9.

### [Third Modification of Wire Traction Mechanism]

A configuration example by the traction wire 9 and the engaging unit 55 will be described as a third modification of the wire traction mechanism, with reference to FIG. 16.

The engaging unit 55 of this modification comprises a small-diameter part 55a and a large-diameter part 55b with a step 55c. A fixing hole 55d is formed in a manner penetrating through the large-diameter part 55b from the distal surface of the small-diameter part 55a. The distal end of the traction wire 9 is inserted into the fixing hole 55d and stopped at a position before piercing through the large-diameter part 55b. The traction wire 9 is fixed by an adhesive at this position, and an epoxy-based adhesive or lead-free stainless solder, for example, is filled in the hole from the bottom side of the large-diameter part 55b to fill up the hole, and is solidified.

In this manner, the distal end of the traction wire 9 is fixed in the engaging unit 55, and the traction wire 9 is not exposed to the outside by filling up the hole. Even if the hole is a through-hole, watertightness can be achieved by fastening and fixing the distal end of the traction wire 9 in the engaging unit 55, and blocking up the through-hole completely by an adhesive, solder, or the like. Even if the traction wire 9 should pierce through the engaging unit 55, letting the distal portion thereof be exposed to the outside, watertightness can still be maintained by covering it with solder or an adhesive having a waterproof function, for example, so as to prevent a contaminated fluid and the like from permeating the exposed part.

### [Fourth Modification of Wire Traction Mechanism]

A configuration example of a joint structure between the tube 14 for the wire and the traction wire 9 hung on the engaging section 21 will be described as a fourth modification of the wire traction mechanism, with reference to FIG. 17.

The fourth modification of the wire traction mechanism is a wire traction mechanism using the above-described engaging section 21 shown in FIG. 12.

The traction wire 9 is fixed to the engaging section 21 fitted into the engaging hole 4b of the swing base 4. Both the traction wire 9 and the small-diameter part 21a are bent backward at a substantially right angle. The tube 14 for the wire which is attached the proximal end of with the coupling unit 31, is inserted in a manner to cover the traction wire 9. The insertion is stopped at a position where the distal end of the tube 14 for the wire is close to the small-diameter part 21a of the engaging section 21. Next, in the state where the traction wire 9 is covered, an adhesive is filled in so as to fill up the clearance between the tube 14 for the wire and the traction wire 9, and the adhesive 21e is shaped in a manner to cover at least the end portion of the small-diameter part 21a and the end portion of the tube 14 for the wire and prevent the traction wire 9 from being exposed.

In this manner, even if the tube 14 for the wire is not adhered to the engaging section 21 directly, a watertight structure can be achieved in which the tube 14 for the wire and the engaging section 21 are connected to each other via an adhesive in a watertight manner and the traction wire 9 is not exposed.

### [Fifth Modification of Wire Traction Mechanism]

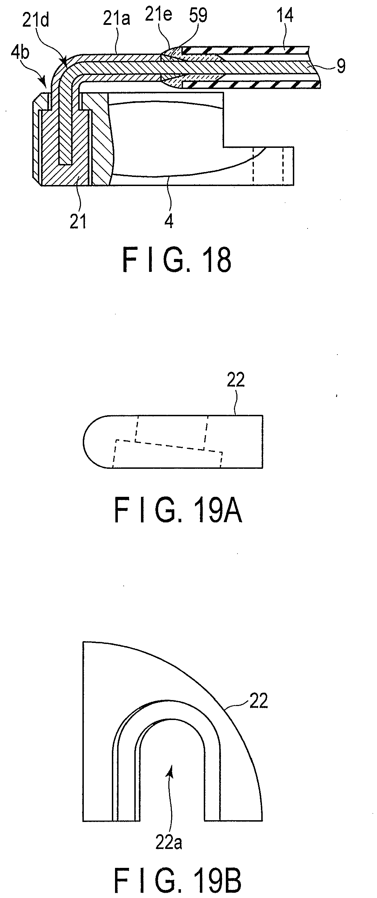

A configuration example of a joint structure between the tube 14 for the wire and the traction wire 9 hung on the engaging section 21 will be described as a fifth modification of the wire traction mechanism, with reference to FIG. 18.

The fifth modification of the wire traction mechanism, like the above-described fourth modification, is a wire traction mechanism using the engaging section 21 shown in FIG. 12. The traction wire 9 is fixed to the engaging section 21 fitted into the engaging hole 4b of the swing base 4 by an adhesive and solder 59.

Next, the tube 14 for the wire covers the traction wire 9, and the insertion is stopped at a position where the covering end portion comes close to the small-diameter part 21a of the engaging section 21 and comes in contact with the solder. Then, an adhesive is filled in so as to cover the tube 14 for the wire and the solder 59 and to fill up the clearance between the tube 14 for the wire and the traction wire 9, and the adhesive 21e is shaped in a manner to cover at least the end portion of the small-diameter part 21a and the end portion of the tube 14 for the wire and prevent the traction wire 9 from being exposed.

According to this modification, the traction wire 9 is kept watertight by fixing the traction wire 9 to the engaging section 21 by the adhesive and the solder 59, and further fixing the tube 14 for the wire to the engaging section 21 while covering it with the adhesive 21e in a manner to include the surface of the solder 59 and make the traction wire 9 watertight.

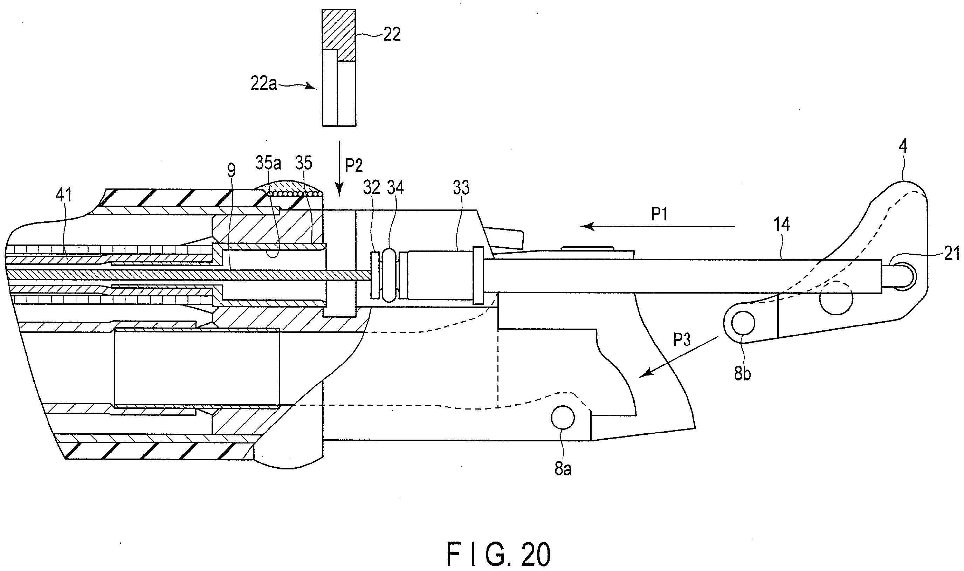

Next, a procedure of assembling the wire traction mechanism will be described with reference to FIGS. 19A, 19B and 20.

FIG. 19A is an external view of the retaining plate 22, seen from the top, which is a guide portion for fixing the tube 14 for the wire and the coupling unit 31. FIG. 19B is an external view of the retaining plate 22 seen from the front. FIG. 20 is a view illustrating a procedure of assembling the wire traction mechanism.

The retaining plate 22 of this embodiment is formed of a metal material or a hard resin member, and has a fan shape with the central angle being 90° and the arc being the same as the outer periphery of the distal end constituting section 2. The retaining plate 22 has a cutout portion formed therein that practically matches the shape of the section where the tube 14 for the wire and the coupling unit 31 are coupled with each other.

This retaining plate 22 fits the cutout portion 22a to the section where the tube 14 for the wire and the coupling unit 31 are coupled with each other, and is adhesively fixed to the base member 3 to thereby prevent the tube 14 for the wire and the coupling unit 31 from coming off.

In addition, sufficiently applying an adhesive to the portion contacting the tube 14 for the wire and the coupling unit 31 as well to fill it up at the time of fixing, as shown in FIG. 1, makes it possible to maintain watertightness so as to prevent a contaminated fluid and the like from permeating the insertion section from a gap.

An assembling procedure will be described with reference to FIGS. 1 and 20.

P1) The traction wire 9 extending out from the coupling unit 31 is shoved in so that it enters the guide tube 41 for the wire from the opening of the cover member 35 attached to the base member 3 and the distal end of the wire reaches the swing base operation section 107 in the operation section 102.

Afterwards, the coupling member 32 attached with the O-ring 34 is inserted and fitted into the pressing portion 35a of the cover member 35. At this time, the edge of the engaging member 33 is located at a predetermined position that abuts on the retaining plate 22. At this time, a position is determined in advance so that a hole 8a to be fitted with the raising shaft 8 of the swing base 4 matches a hole 8b to be fitted with the raising shaft 8 provided to the base member 3. A temporary joint can be made depending on the case.

P2) The silicone-based adhesive 23 is applied to the inner surface of the cutout portion 22a and the portion of the retaining plate 22 joining the base member 3, to fit and fix the retaining plate 22 to the base member 3.

P3) After matching the hole 8a and the hole 8b in a manner to overlap each other, the raising shaft 8 passing through the hole 8a is fixed to the hole 8b.

### [Third Embodiment]

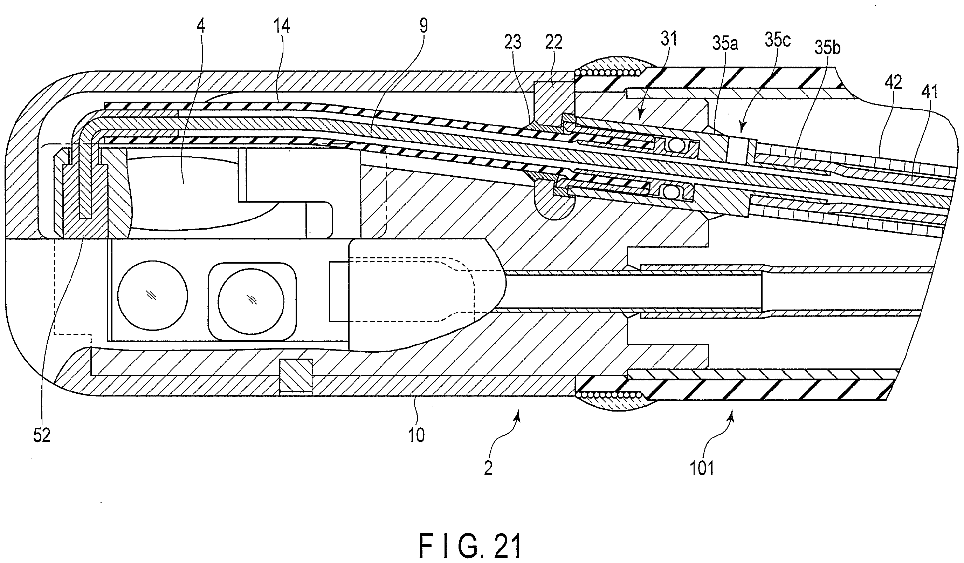

A third embodiment will be described with reference to FIG. 21.

FIG. 21 is a cross-sectional view showing a cross-section structure, seen from the top, of a distal portion of the insertion device in which the swing base of the distal end constituting section of the insertion device is laid down. In the description of this embodiment, the structural parts equivalent to those of the first embodiment are identified by the same reference symbols as those used for the first embodiment, and detailed explanations thereof are omitted.

The end portion of the guide tube 41 for the wire is opened in the operation section 102, as described above. In this embodiment, a second opening 35c, which opens in the insertion section 101 on the proximal side of the distal end constituting section, is provided near the pressing portion 35a of the cover member 35.

In a washing step after finishing examination and treatment, whether or not there is a water leak in the endoscope is confirmed without fail. As the method to confirm this, the endoscope is sunk into water with the inside of the endoscope pressurized, to confirm that there are no bubbles coming out of the endoscope. Namely, if there are bubbles coming out of the tube 14 for the wire, the endoscope is broken for some reason.

However, even if the inside of the operation section 102 is pressurized, it takes some time for the inside of the tube 14 for the wire to be pressurized because the insertion section 101 is long, and the length from the guide tube 41 for the wire to the tube 14 for the wire is large.

In this embodiment, the second opening 35c is opened at a position that is close to the tube 14 for the wire and in which a contaminated fluid and the like do not enter from the outside, i.e. , in the vicinity of the pressing portion 35a of the cover member 35 in the insertion section 101. By opening at this position, a gas is sent into the tube 14 for the wire immediately after starting pressurization of the inside of the endoscope. Thus, in the case where there is a hole, a crack, or the like in the tube 14 for the wire, it can be checked immediately. Accordingly, a water leak is less likely to be overlooked, a negative influence of liquid on an electronic device by mistake can also be prevented, and the problem can be solved simply by replacing the component.

### [Fourth Embodiment]

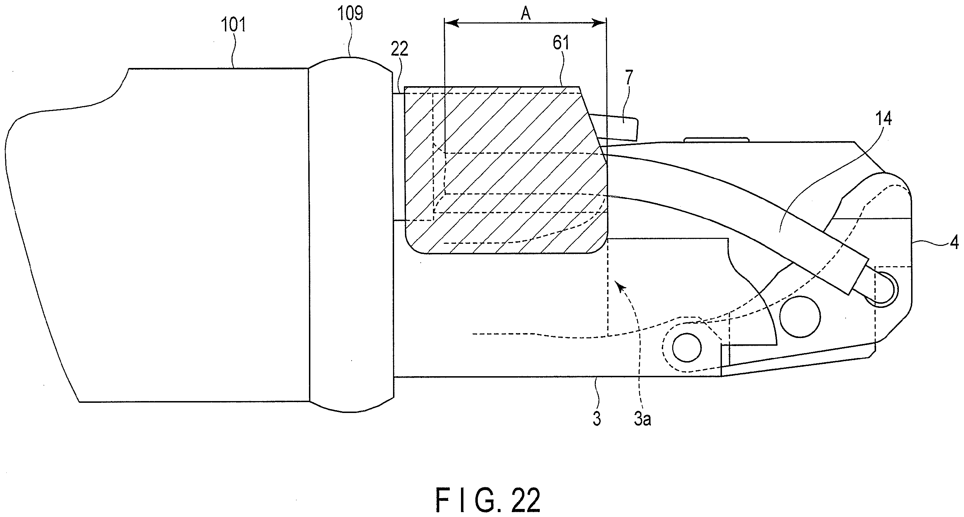

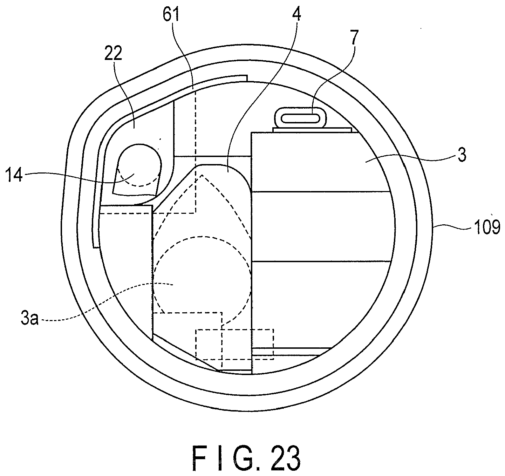

A fourth embodiment will be described with reference to FIGS. 22 and 23.

FIG. 22 is a view of a configuration, seen from the lateral side, in which a cover is provided to the distal end constituting section of the insertion device. FIG. 23 is a view of the configuration, seen from the distal side, in which the cover is provided to the distal end constituting section of the insertion device. In the description of this embodiment, the structural parts equivalent to those of the first embodiment are identified by the same reference symbols as those used for the first embodiment, and detailed explanations thereof are omitted.

As shown in FIG. 22, a tube cover 61 is provided that has a length ranging from the channel opening 3a in the base member 3 of the distal end constituting section to the retaining plate 22 and including the above-described distance A. The tube cover 61 covers the tube 14 for the wire, ranging from the side surface to the top surface in a manner to isolate the tube 14, as show in FIG. 23.

Providing such tube cover 61 prevents the wrinkle 14a generated in the tube cover 61 at the time of raising the swing base 4 from contacting the external elements, as shown in FIG. 4, and thereby prevents a treatment target from being held or engulfed by the wrinkle.

As described above, the insertion device of this embodiment has the features and effects described below.

In the long insertion device, the entire connecting part of the end portion of the traction wire coupled with the swing base in the distal end constituting section is covered with a tubular elastic member in a watertight manner, and the elastic member is fixed at a position that is away from the opening of the treatment tool channel hole toward the proximal side by a predetermined distance in the longitudinal axis direction. This prevents the traction wire from being exposed to the outside and prevents a contaminated fluid and the like from adhering thereto during treatment. Accommodating the successive wrinkles generated in the elastic member shrunk at the time of raising the swing base in the space created at a predetermined distance prevents the wrinkles from sticking to each other. The amplitude of the wrinkle generated can be set by changing the thickness of the elastic member depending on the area where the elastic member is disposed, and the engulfment in the elastic member or the damage thereof can be avoided by decreasing the amplitude of the wrinkle in the area adjacent to the moving device such as the swing base 4.

In addition, by providing an opening at a position that is close to the part of the tubular elastic member that is exposed in the distal end constituting section and where the tubular elastic member is not exposed to the outside in the inside of the insertion device, etc., and pressurizing the inside of the insertion device, the state, or position of damage can be identified easily from a gas leak from the elastic member exposed to the outside.

## Claims

1. An insertion device **characterized by** comprising:
an operating portion that is operable and provided to an insertion section inserted into a lumen;
a long traction member that is connected to the operating portion and moves in a longitudinal axis direction of the insertion section, to operate the operating portion;
a distal end constituting section that is provided on a distal side of the insertion section and comprises: a treatment tool channel hole that communicates with a treatment tool insertion tube disposed in the insertion section; and an introducing hole that is provided away from an opening of the treatment tool channel hole toward a proximal side of the insertion section and guides the traction member; and
a tubular elastic member in which the traction member is disposed, one end being connected to the operating portion in a watertight manner so as to prevent the traction member connected to the operating portion from being exposed to an external part, the other end being connected to the introducing hole in a watertight manner.

2. The insertion device according to claim 1, **characterized in that** an end portion of the elastic member is fitted into the introducing hole, so that the elastic member is fixed to the distal end constituting section in a watertight manner.

3. The insertion device according to claim 1, **characterized in that** an opening on an end surface of the introducing hole is provided away from the opening of the treatment tool channel hole toward the proximal side of the insertion section at a predetermined distance.

4. The insertion device according to claim 1, **characterized in that** the distal end constituting section is configured to support at least a part of the operating portion at a position facing the opening of the treatment tool channel hole.

5. The insertion device according to claim 4, **characterized in that** the operating portion includes a swing base that swings around a predetermined supporting portion provided to the distal end constituting section by traction force from the traction member, and changes a direction of a treatment tool protruding from the opening of the treatment tool channel hole.

6. The insertion device according to claim 1, **characterized in that** the elastic member is configured so that a side thereof that is connected to the introducing hole is more likely to undergo compression deformation in a longitudinal direction than a side thereof that is connected to the operating portion.

7. The insertion device according to claim 6, **characterized in that** the elastic member is configured so that a part thereof that is always positioned in a length from an opening of an end surface of the introducing hole to the opening of the treatment tool channel hole is more likely to undergo compression deformation in a longitudinal axis direction to create successive wrinkles than a part thereof that is always positioned in a length from the opening of the treatment tool channel hole to the operating portion.

8. The insertion device according to claim 1, comprising a distal end cover that covers a perimeter of the operating portion, the traction member, the distal end constituting section and the elastic member and is attached to and detached from the insertion section.

9. An endoscope **characterized by** including the insertion device according to claim 1.
